# EUROPÄISCHE PATENTSCHRIFT

(11) **EP 2 688 469 B1**
(45) Veröffentlichungstag und Bekanntmachung des Hinweises auf die Patenterteilung: **04.03.2015**
(21) Anmeldenummer: 12709272.4
(22) Anmeldetag: 31.01.2012
(51) Int. Cl.: A61B 5/0478, A61B 5/0408, A61B 5/00

(54) **VORRICHTUNG ZUM ANLEGEN VON ELEKTRODENANORDNUNGEN**
DEVICE FOR APPLYING ELECTRODE ASSEMBLIES
DISPOSITIF DE POSE D'AGENCEMENTS D'ÉLECTRODES

(30) Priorität: 21.03.2011 AT 4022011
(43) Veröffentlichungstag der Anmeldung: 29.01.2014
(60) Teilanmeldung: 14199538.1
(73) Patentinhaber: Guger, Christoph, 4533 Piberbach (AT); Edlinger, Günter, 8020 Graz (AT)
(72) Erfinder: Guger, Christoph, 4533 Piberbach (AT); Edlinger, Günter, 8020 Graz (AT)
(74) Vertreter: Wildhack & Jellinek
(86) Internationale Anmeldenummer: PCT/AT2012/000014
(87) Internationale Veröffentlichungsnummer: WO 2012/126021

(56) Entgegenhaltungen:
- EP-A1- 1 767 147
- WO-A1-2009/134763
- WO-A2-2008/109699
- US-A- 1 099 062
- US-A1- 2011 046 503

## Beschreibung

Die Erfindung betrifft eine Vorrichtung zum Anlegen von Elektrodenanordnungen an die Hautoberfläche eines tierischen oder menschlichen Lebewesens gemäß dem unabhängigen Patentanspruch 1.

Im folgenden wird das Lebewesen - ob Mensch oder Tier -, an dessen Haut Spannungen oder Spannungsdifferenzen zu bestimmen sind, als Patient bezeichnet.

Erfindungsgemäße Elektrodenanordnungen werden insbesondere im medizinischen Bereich zur Vermessung von Hirnströmen oder der Herzaktivität eingesetzt:
Bei der Verwendung von Trockenelektroden zur Detektion von elektrischen Signalen im Inneren des Körpers des Patienten stellt die korrekte Positionierung der Elektroden eine wesentliche Notwendigkeit dar. Gemäß dem Stand der Technik wie zum Beispiel in EP 1767147, WO 2008/109699 oder WO 2009/134763 gegeben, werden sogenannte Spiders verwendet, die die einzelnen Elektroden bzw. Elektrodenanordnungen auf dem zu vermessenden Körperteil des Patienten fixieren und so für einen möglichst guten Halt und einen einwandfreien Kontakt der Elektroden sorgen.

Spiders weisen eine Vielzahl von Armen auf, an deren Enden die einzelnen Elektroden angeordnet sind. Problematisch ist hierbei, dass die Position und Ausrichtung der einzelnen Elektroden an die jeweilige Form des Patienten angepasst werden muss und somit sehr viele unterschiedliche Einstellungsschritte vorgenommen werden müssen, um die Elektroden an den Patienten anzupassen. Weiters besteht das Problem, dass die Elektroden nur schlecht mit dem Patienten in Kontakt stehen und leicht verrutschen können, wenn sich der Patient bewegt.

Aufgabe der Erfindung ist es, eine vereinfachte Anpassung der einzelnen Elektroden an die Körperform, insbesondere an die Kopfform, des Patienten zu ermöglichen und somit eine raschere Abfolge bei der Untersuchung von mehreren Patienten zu ermöglichen.

Die Erfindung löst diese Aufgabe mit einer Vorrichtung zum Anlegen an die Hautoberfläche eines tierischen oder menschlichen Lebewesens mit den Merkmalen des Kennzeichens des unabhängigen Patentanspruchs 1.

Bei einer Vorrichtung mit einer Anzahl von Elektrodenanordnungen, die an die Hautoberfläche eines tierischen oder menschlichen Lebewesens anlegbar sind und mit denen Spannungen und Ströme von der Hautoberfläche abgreifbar sind und mit einem mit einem flächigem oder folienartigem Formteil gebildeten flexiblen, insbesondere dehnbaren, Halteelement, ist erfindungsgemäß vorgesehen, dass die Elektrodenanordnungen einen Grundkörper sowie einer Anzahl von vom Grundkörper nach derselben Richtung abstehenden Stiftelektroden aufweisen, dass die Elektrodenanordnungen mit dem Halteelement befestigt sind, und dass der Grundkörper der jeweiligen Elektrodenanordnung mit dem Halteelement verbunden ist, wobei die Stiftelektroden sämtlicher Elektrodenanordnungen nach derselben Richtung abstehen. Hierdurch werden eine einfache Positionierung der Elektroden sowie eine einfache Anpassung der Lage der Elektroden an den jeweiligen Patienten ermöglicht.

Um die Anpassung an spezielle Körperteile zu verbessern, kann vorgesehen sein, dass das Halteelement als Haube oder als Manschette ausgebildet ist.

Ferner kann vorgesehen sein, dass im Grundkörper zumindest bei einer der Elektrodenanordnungen, insbesondere bei allen Elektrodenanordnungen, eine durchgängige Ausnehmung vorgesehen ist, durch die bei Anliegen der Stiftelektroden an der Hautoberfläche ein den Kontaktwiderstand zwischen der Hautoberfläche und den Stiftelektroden verringerndes Gel auf die Hautoberfläche aufbringbar ist.

Hierdurch ist es möglich, einen verbesserten Kontakt der Stiftelektroden mit der Hautoberfläche des Patienten zu erreichen, wobei der Einfluss von Bewegungen des Patienten bei der Messung minimiert ist. Zudem besteht der Vorteil, dass bei ausreichend gutem Kontakt die Verwendung von Gel unterbleiben kann, was eine Verunreinigung des jeweiligen Patienten mit dem Gel vermeidet. Kann die Verwendung von Gel aufgrund ausreichenden Kontakts entfallen, ist auch ein zeitaufwendiges Aufbringen von Gel nicht erforderlich.

Zum leichteren Einbringen von Gel an die jeweiligen Hautstellen während der Messung kann vorgesehen sein, dass das Halteelement Ausnehmungen aufweist, deren jeweilige Position an die Position jeweils einer der Ausnehmungen im Grundkörper angepasst ist, sodass Gel durch die Ausnehmungen im Halteelement und durch die Ausnehmung im Grundkörper auf die Hautoberfläche aufbringbar ist.

Um ein besonders gutes Anliegen am Körper des Patienten, insbesondere bei längeren Messungen, zu erreichen, kann vorgesehen sein, dass der Grundkörper flach oder plattenförmig mit zwei einander gegenüberliegenden Oberflächenbereichen ausgebildet ist, wobei Ausnehmung durchgängig ausgebildet ist und die Berandung der Ausnehmung die beiden Oberflächenbereiche miteinander verbindet.

Zum vorteilhaften Aufbringen des Gels auf den Stiftelektroden sowie zur Verringerung des Kontaktwiderstands zwischen den Stiftelektroden und der Hautoberfläche kann vorgesehen sein, dass die Stiftelektroden am Rand der Ausnehmung an der Grundplatte befestigt sind.

Zur optimalen Verteilung des eingebrachten Gels kann vorgesehen sein, dass die Ausnehmung kreisförmig ausgebildet ist.

Ferner kann zur Verbesserung der Auflagestabilität der Elektrodenanordnung auf der Haut des Patienten vorgesehen sein, dass je zwei benachbart angeordnete Stiftelektroden jeweils voneinander denselben Abstand haben.

Um eine verbesserte Kontaktierung der Stiftelektroden zu erzielen und Übertragungsfehler zu verringern, ist vorgesehen, dass die Stiftelektroden auf einem gemeinsamen Elektrodenring angeordnet sind, wobei der Elektrodenring vorzugsweise aus demselben Material besteht wie die Stiftelektroden.

Zur vorteilhaften Durchdringung der an der Hautoberfläche befindlichen Haare sowie zur Erreichung einer vorteilhaften Auflagestabilität der Elektrodenanordnung auf der Haut des Patienten kann vorgesehen sein, dass die Stiftelektroden eine Höhe und/oder Anzahl und/oder Länge und/oder Anordnung aufweisen, dass sie eine auf der Hautoberfläche befindliche Behaarung durchdringen können, jedoch eine stabile Auflage gewährleisten, und/oder dass die Höhe der Stiftelektroden zwischen 1 mm und 30 mm liegt.

Zum vorteilhaften Verteilen des Gels auf der Hautoberfläche kann vorgesehen sein, dass die von der Ausnehmung ausgenommene Fläche des Grundkörpers 0,785 mm² bis 700 mm² beträgt und/oder der Radius der Ausnehmung zwischen 1 mm und 30 mm beträgt.

Zur Verbesserung des Kontakts zwischen der Hautoberfläche und den Stiftelektroden sowie zur Verbesserung der mechanischen Auflagestabilität kann vorgesehen sein, dass der Durchmesser der Stiftelektroden derart gewählt ist, dass die Stiftelektrode durch die Behaarung der Hautoberfläche hindurchdringen kann, bei einem einen elektrischen Kontakt ermöglichenden Anpressdruck die Hautoberfläche jedoch nicht verletzt, und/oder dass der Durchmesser der Stiftelektroden zwischen 0,5 mm und 5 mm liegt.

Zur Verbesserung des Kontakts zwischen der Hautoberfläche und den Stiftelektroden sowie zur Vermeidung der Verletzung des Patienten kann vorgesehen sein, dass das dem Grundkörper ferne Ende der Stiftelektroden halbkugelförmig ausgebildet ist.

Zur dauerhaften elektrischen Kontaktierung sowie zur Verringerung des Kontaktwiderstands und der Kontaktspannung kann vorgesehen sein, dass die Stiftelektroden hochleitfähig sind und insbesondere aus Gold, TiN, IrO₂ oder einer Gold und/oder TiN, IrO₂ enthaltenden Legierung bestehen.

Um elektrische Störungen, etwa durch elektromagnetische Interferenzen, zu vermeiden, kann vorgesehen sein, dass eine Verstärkeranordnung vorgesehen ist und die Stiftelektroden an die Verstärkeranordnung angeschlossen sind. Hierdurch wird eine Verwendung selbst bei hohem Hautwiderstand möglich.

Bei der Anordnung der Verstärkerschaltung auf dem Grundkörper ist eine vereinfachte Kontaktierung der Elektrode möglich.

Bei einer maximalen Entfernung der Verstärkeranordnung von weniger als 20 mm zur nächstgelegenen Stiftelektrode ist eine optimale Verstärkung mit minimalem Rauschen möglich.

Zur einfachen Auswechselbarkeit von Elektroden sowie zur Verringerung der Anzahl von Verstärkeranordnungen kann vorgesehen sein, dass die Verstärkeranordnung auf einem mit dem Grundkörper elektrisch und mechanisch, insbesondere über Rastverbindungen, lösbar verbindbaren separaten weiteren Grundkörper angeordnet ist.

Um einen einfachen Aufbau der erfindungsgemäßen Vorrichtung zu gewährleisten und eine rasche Auswechslung fehlerhafter Elektroden sowie auf weiteren Grundkörpern befindlichen Verstärkern zu ermöglichen, kann vorgesehen sein, dass das Halteelement eine Anzahl von Ausnehmungen aufweist und die Rastverbindungen die Ausnehmungen des Halteelements durchsetzen

Die Erfindung wird ohne Einschränkung des allgemeinen erfinderischen Gedankens anhand mehrerer Beispiele unter Zuhilfenahme der folgenden Figuren beschrieben.

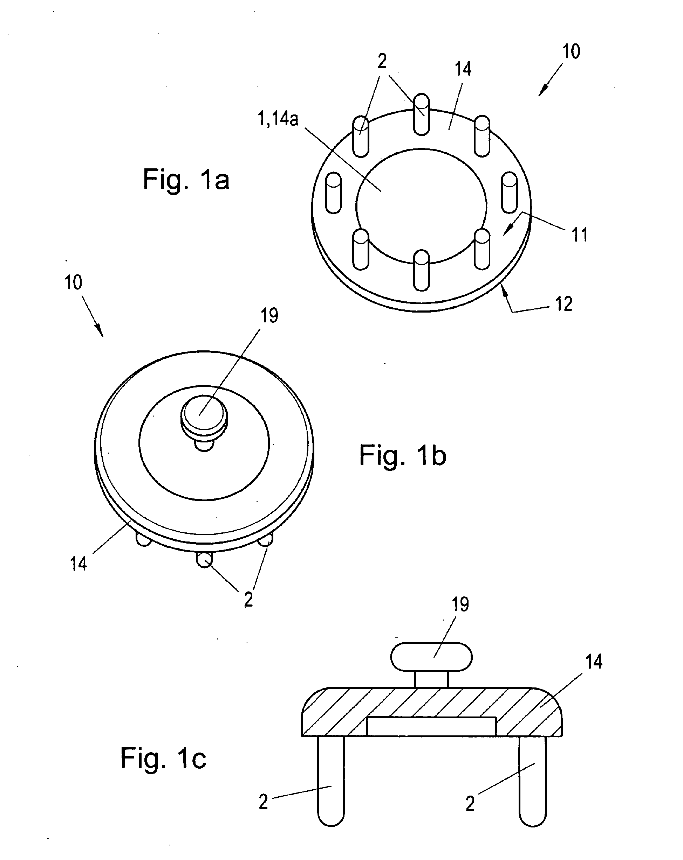

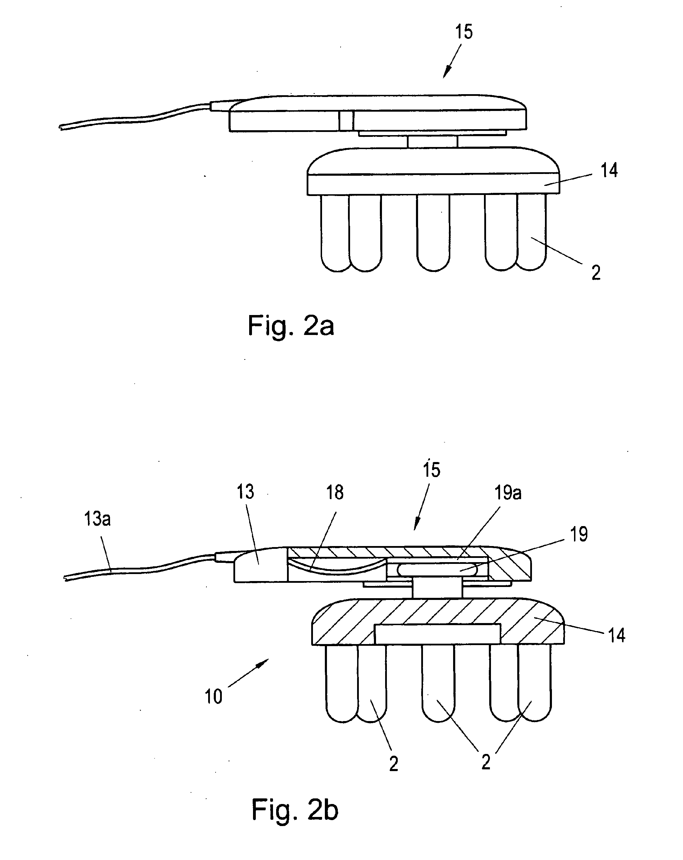

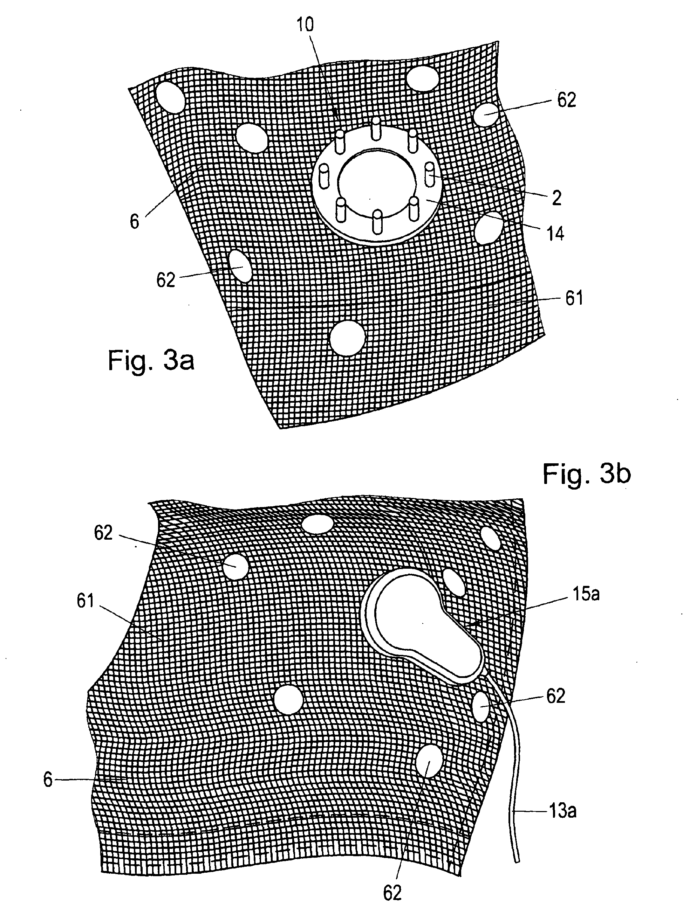

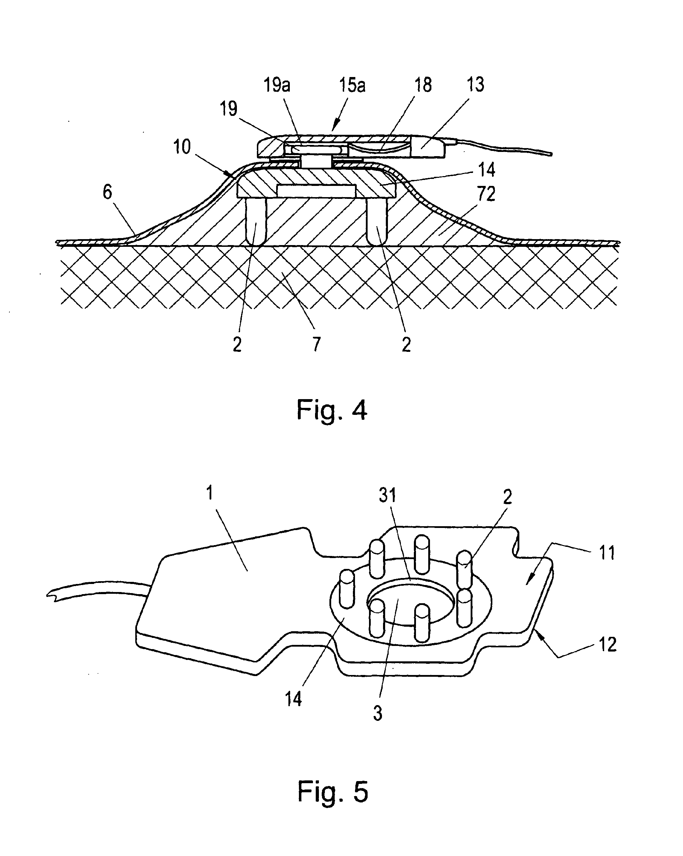

**Fig. 1a und 1b** zeigen zwei Abbildungen einer Elektrodenanordnung aus unterschiedlichen Ansichten. **Fig. 1c** zeigt die Elektrodenanordnung im Querschnitt. **Fig. 2a** zeigt eine separat auf einem weiteren Grundkörper ausgebildete Verstärkerschaltung, die mit der in **Fig. 1a bis 1c** dargestellten Elektrodenanordnung kontaktiert ist, in Seitenansicht. **Fig. 2b** zeigt die in **Fig. 2a** dargestellte Anordnung im Querschnitt. **Fig. 3a** zeigt eine Haube mit einer Elektrodenanordnung und innen. **Fig. 3b** zeigt die in **Fig. 3a** dargestellte Haube von außen. **Fig. 4** zeigt einen Ausschnitt einer in Anlage zu einem Patienten stehenden Vorrichtung zum Anlegen an die Hautoberfläche im Querschnitt.

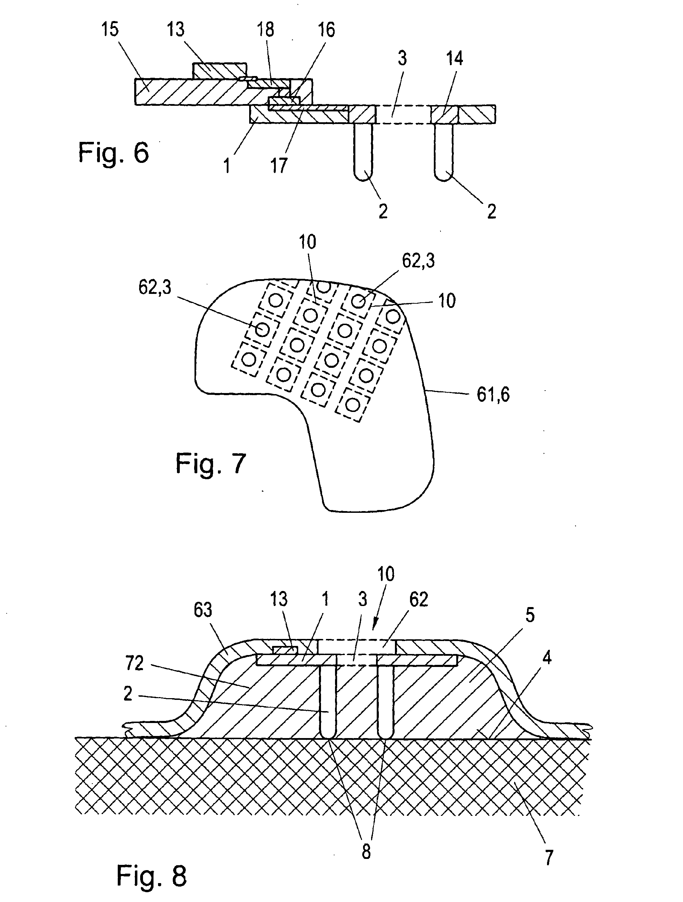

**Fig. 5** zeigt eine alternative erfindungsgemäße Elektrodenanordnung. **Fig. 8** zeigt eine separat auf einer Platine ausgebildete Verstärkerschaltung, die mit einer Elektrodenanordnung kontaktiert ist. **Fig. 7** zeigt eine Haube mit einer Anzahl von Elektrodenanordnungen. **Fig. 8** zeigt einen Ausschnitt einer in Anlage zu einem Patienten stehenden Vorrichtung zum Anlegen an die Hautoberfläche im Querschnitt.

In den **Fig. 1a bis 1c** ist eine Ausführungsform einer Elektrodenanordnung 10 dargestellt. Diese umfasst einen als Platine ausgebildeten Grundkörper 1. Der Grundkörper 1 ist im wesentlichen flach ausgebildet und weist eine Dicke von 1,2 mm auf. Die beiden einander gegenüberliegenden Oberflächenbereiche 11, 12 des Grundkörpers 1 sind eben ausgebildet und liegen zueinander parallel. Im Grundkörper 1 ist kreisförmig ausgebildet.

Im Bereich des Randes des kreisförmigen Grundkörpers 1 steht eine Anzahl von acht Stiftelektroden 2 von einem der Oberflächenbereiche 11 ab. Die Anzahl der Stiftelektroden 2 beträgt vorteilhafterweise mehr als drei, damit eine mechanisch besonders stabile Auflage auf der Hautoberfläche 4 des Patienten 7 (**Fig. 4****,** **8**) möglich ist. Andererseits ist die Anzahl der Stiftelektroden 2 so gewählt, dass zwischen den Stiftelektroden 2 ausreichender Abstand für die Aufnahme von Haaren besteht, damit nicht einzelne Haare des Patienten 7 zwischen den Stiftelektroden 2 und der Hautoberfläche 4 eingeklemmt werden. Der Abstand je zweier benachbarter Stiftelektroden 2 entspricht jeweils etwa dem doppelten des Durchmessers einer Stiftelektrode 2.

Im vorliegenden Ausführungsbeispiel haben die Stiftelektroden 2 eine Länge von 7 mm und einen Durchmesser von 2,5 mm. Die von dem Grundkörper 1 entfernt liegenden Enden der Stiftelektroden 2 weisen Halbkugelform auf.

Die Stiftelektroden 2 bestehen im vorliegenden Ausführungsbeispiel aus reinem Gold mit möglichst geringen Verunreinigungen.

Alternativ können jedoch auch unterschiedliche Legierungen verwendet werden, wobei die Stiftelektroden 2 vorteilhaft einen geringen elektrischer Widerstand aufweisen. Weiters ist es vorteilhaft, wenn die Stiftelektroden 2 eine hohe mechanische Stabilität, insbesondere Biege- und Bruchfestigkeit aufweisen, wie es etwa bei TiN- oder IrO₂ - Legierungen der Fall ist.

Der Grundkörper 1 weist in seinem Randbereich sowie in seinem von den Stiftelektroden 2 abgewandten Bereich einen aus einer Goldlegierung bestehenden, elektrisch hochleitfähigen Elektrodenring 14 auf, auf den die Stiftelektroden 2 aufgelötet sind. Der Elektrodenring 14 bildet im vorliegenden Ausführungsbeispiel einen Teil des Grundkörpers 1 und weist die Form einer hohlzylindrischen Schicht mit einer Schichtdicke von 1,2mm, einem Innenradius von 11mm und einem Außenradius von 17 mm auf. Der im Inneren des Grundkörpers 1 liegende und vom Elektrodenring 14 unmrandete Teil 14a ist nicht leitfähig.

Alternativ kann der gesamte Grundkörper 1 aus hochleitfähigem Material, insbesondere auch aus Elektrodenmaterial bestehen.

Alternativ können die Stiftelektroden 2 auch mit dem Elektrodenring 14 verschweißt oder sonst elektrisch leitend und mechanisch verbunden sein.

Auf dem dem Oberflächenbereich 11, von dem die Stiftelektroden abstehen, gegenüberliegenden Oberflächenbereich 12 ist ein Verbindungsteil 19 vorgesehen, der mit dem Elektrodenring 14 und somit mit den einzelnen Stiftelektroden 2 leitfähig verbunden ist. Im vorliegenden Fall ist der Verbindungsteil 19 als Rastverbindung ausgebildet, der lösbar mit einem weiteren Verbindungsteil 19a (Fig. 2a, 2b) in Verbindung bringbar ist. Der Verbindungsteil 19 ist ebenfalls aus einem gut leitfähigen Material ausgebildet, um eine vorteilhafte Weiterleitung der von den Stiftelektroden 2 ermittelten Signale zu ermöglichen.

In den **Fig. 2a und 2b** ist die in **Fig. 1a bis 1c** dargestellte Elektrodenanordnung 10 über den Verbindungsteil 19 mit einem weiteren Grundkörper 15 elektrisch und mechanisch verbunden, der seinerseits einen weiteren Verbindungsteil 19a aufweist, der mit dem Verbindungsteil der Elektrodenanordnung elektrisch leitend und mechanisch in Verbindung bringbar ist. Es wird somit eine zweiteilige Elektrodenanordnung dargestellt, wobei der dargestellte Grundkörper 1 sowie der Aufbau der Stiftelektroden 2 der in **Fig. 1a bis 1c** dargestellten Ausführungsform entspricht. Auf dem weiteren Grundkörper 15 ist eine Verstärkeranordnung 13 ausgebildet. Der Grundkörper 1 und der weitere Grundkörper 15 sind über die jeweiligen Verbindungsteile 19, 19a miteinander elektrisch leitend verbunden, wobei auf Seiten des weiteren Grundkörpers 15 eine Verbindungsleitung 18 zwischen dem weiteren Verbindungsteil 19a und einem Verstärker 13 angeordnet ist. Die Rastverbindung zwischen dem Grundkörper 1 und dem weiteren Grundkörper 15 ist mechanisch zerstörungsfrei lösbar und wiederherstellbar. Das Ausgangssignal des Verstärkers 13 wird über ein Kabel 13a an eine Datenverarbeitungseinheit weitergeleitet.

In den **Fig. 3a, 3b** ist ein Halteelement 6 in Form einer Haube 61 von der Innenseite sowie von der Außenseite gesehen dargestellt, wobei lediglich eine einzige Elektrodenanordnung 10 bzw. ein einziger weiterer Grundkörper 15 dargestellt ist. Die Haube 61 ist zum Anlegen an die Hautoberfläche 4 am Kopf eines Patienten 7 ausgebildet. Die Elektrodenanordnungen 10 entsprechen im wesentlichen den in den **Fig. 2a, 2b** dargestellten Elektrodenanordnungen 10. Der Grundkörper 1 der Elektrodenanordnungen 10 liegt an der Haube 61 innen an, wobei der weitere Oberflächenbereich 12 mit der Innenfläche der Haube 61 in physischem Kontakt steht. Die Stiftelektroden 2 weisen in das Innere der Haube 61. Die Haube 61 weist in den Bereichen, in denen sich Rastverbindungen 16 der Elektrodenanordnungen 10 befinden, Ausnehmungen 62 auf, die eine unmittelbare Kontaktierung mit einem weiteren Grundkörper 15 ermöglichen (**Fig. 4**), sodass die Hautoberfläche des Patienten unmittelbar durch die Haube 61 hindurch elektrisch kontaktierbar ist.

Der weitere Grundkörper 15 befindet sich an der Außenseite der Haube 61, wobei diejenige Seite, nach der der weitere Verbindungsteil 19a gerichtet ist, der Haube 61 zugewandt ist und mit dem die Ausnehmung 62 der Haube durchsetzenden Verbindungsteil 19 in elektrischem und mechanischem Kontakt steht.

In **Fig. 4** ist die in **Fig. 3a und 3b** dargestellte Ausführungsform der Erfindung im Querschnitt dargestellt. Die Haube 62 weist, wie auch in **Fig. 3a und 3b** eine Anzahl von an ihrer Innenfläche angeordneten Elektrodenanordnungen 10 auf. Die Ausnehmungen 62 der Manschette 63 sind von den Verbindungsteilen 19, 19a des Grundkörpers 1 der Elektrodenanordnung 10 bzw. des weiteren Grundkörpers durchsetzt.

Schraffiert dargestellt ist ein von Haaren gefüllter Zwischenbereich, im folgenden Haarbereich 72 genannt, zwischen der Hautoberfläche 4 und dem Grundkörper 1. Der Grundkörper 1 und der Hautoberfläche 4 des Patienten 7 werden einerseits durch die Haare, andererseits durch die Stiftelektroden 2 auf Abstand gehalten.

In **Fig. 5** ist eine alternative Ausführungsform einer Elektrodenanordnung 10 dargestellt. Diese umfasst einen als Platine ausgebildeten Grundkörper 1. Der Grundkörper 1 ist im wesentlichen flach ausgebildet und weist eine Dicke von 1,2 mm auf. Die beiden einander gegenüberliegenden Oberflächenbereiche 11, 12 des Grundkörpers 1 sind eben ausgebildet und liegen zueinander parallel. Im Grundkörper 1 ist eine kreisförmige Ausnehmung 3 ausgebildet. In diesem bevorzugten Ausführungsbeispiel der Erfindung beträgt der Durchmesser der Ausnehmung 5 mm, der Radius somit 2.5 mm. Die Ausnehmung 3 bzw. die ausgenommene Fläche des Grundkörpers 1 weist eine Fläche von etwa 50 mm² auf.

Im Bereich des Randes der Ausnehmung 3 steht eine Anzahl von sieben Stiftelektroden 2 von einem der Oberflächenbereiche 11 ab. Die Anzahl der Stiftelektroden 2 beträgt vorteilhafterweise mehr als drei, damit eine mechanisch besonders stabile Auflage auf der Hautoberfläche 4 (**Fig. 8**) des Patienten 7 (**Fig. 8**) möglich ist. Andererseits ist die Anzahl der Stiftelektroden 2 so gewählt, dass zwischen den Stiftelektroden 2 ausreichender Abstand für die Aufnahme von Haaren besteht, damit nicht einzelne Haare des Patienten 7 zwischen den Stiftelektroden 2 und der Hautoberfläche 4 eingeklemmt werden. Der Abstand je zweier benachbarter Stiftelektroden 2 entspricht jeweils etwa dem Durchmesser einer Stiftelektrode 2.

Im vorliegenden Ausführungsbeispiel haben die Stiftelektroden 2 eine Länge von 7 mm und einen Durchmesser von 2,5 mm. Die von dem Grundkörper 1 entfernt liegenden Enden der Stiftelektroden 2 weisen Halbkugelform auf.

Die Stiftelektroden bestehen im vorliegenden Ausführungsbeispiel aus reinem Gold mit möglichst geringen Verunreinigungen.

Alternativ können jedoch auch unterschiedliche Legierungen verwendet werden, wobei die Stiftelektroden 2 vorteilhaft einen geringen elektrischer Widerstand aufweisen. Weiters ist es vorteilhaft, wenn die Stiftelektroden 2 eine hohe mechanische Stabilität, insbesondere holen Biege- und Bruchfestigkeit, aufweisen, wie es etwa bei TiN-Legierungen der Fall ist.

Der Grundkörper 1 weist im Bereich der Ausnehmung 3 einen aus einer Goldlegierung bestehenden, elektrisch hochleitfähigen Elektrodenring 14 auf, auf den die Stiftelektroden 2 aufgelötet sind. Der Elektrodenring 14 weist einen quadratischen Querschnitt von 10 mm x 10 mm auf. Der Radius der Ausnehmung 3 im Inneren des Elektrodenrings 14 beträgt 5 mm, die Fläche der Ausnehmung beträgt somit 50 mm². Alternativ können die Stiftelektroden 2 auch mit dem Elektrodenring 14 verschweißt oder sonst elektrisch leitend und mechanisch verbunden sein. Der Elektrodenring 14 durchsetzt den Grundkörper 1, wobei die in Richtung der zentralen Ausnehmung des Elektrodenrings 14 weisende Oberfläche eine zu den Oberflächenbereichen 11, 12 normal stehende zylindermantelförmige Berandung 31 der Ausnehmung 3 bildet. Der Elektrodenring 14 ist über eine auf dem in **Fig. 5** nicht dargestellten weiteren Oberflächenbereich 12 befindliche hochleitfähige elektrische Verbindungsleitung mit einer Verstärkeranordnung 13 (**Fig. 8**) verbunden. Diese elektrische Verbindungsleitung besteht in diesem Ausführungsbeispiel vorteilhafterweise aus dem Elektrodenmaterial der Stiftelektroden 2.

Diese Verstärkeranordnung 13 weist einen geringen Abstand zu den einzelnen Stiftelektroden 2 auf und ist im vorliegenden Ausführungsbeispiel zur Verringerung von elektromagnetischen Störungen 4 mm von nächstgelegenen Stiftelektrode 2 entfernt.

**Fig. 6** zeigt eine alternative zweiteilige Elektrodenanordnung 10, wobei der dargestellte Grundkörper 1 sowie der Aufbau der Stiftelektroden 2 der in **Fig. 5** dargestellten Ausführungsform entspricht. Im Unterschied zu der in **Fig. 5** dargestellten Ausführungsform weist der Grundkörper 1 in der in **Fig. 6** dargestellten Alternative einen weiteren Grundkörper 15 auf, auf dem die Verstärkeranordnung 13 ausgebildet ist. Der Grundkörper 1 und der weitere Grundkörper 15 sind über Verbindungsleitungen 17, 18 auf dem Grundkörper 1 sowie dem weiteren Grundkörper 15 und über eine Rastverbindung 16 elektrisch leitend miteinander verbunden. Die Rastverbindung 16 ermöglich zudem die lösbare mechanische Verbindung des Grundkörpers 1 und des weiteren Grundkörpers 15.

Die elektrisch Verbindungsleitungen 17, 18 sind im vorliegenden Ausführungsbeispiel als oberflächliche Leiterschichten auf den Grundkörpern 1, 15 ausgebildet.

In **Fig. 7** ist ein Halteelement 6 in Form einer Haube 61 zum Anlegen an den Hautoberfläche 4 am Kopf eines Patienten 7 dargestellt. Die Elektrodenanordnungen 10 entsprechen im wesentlichen den in **Fig. 5** dargestellten Elektrodenanordnungen 10. Die Grundkörper 1 der Elektrodenanordnungen 10 liegen an der Haube 61 innen an, wobei der weitere Oberflächenbereich 12 mit der Innenfläche der Haube 61 in mechanischem Kontakt steht. Die Stiftelektroden 2 weisen in das Innere der Haube 61. Die Haube 61 weist in den Bereichen, in denen sich die Ausnehmungen 3 der Elektrodenanordnungen 10 befinden, Ausnehmungen 62 auf, sodass ein Gel 5 (**Fig. 8**) unmittelbar von außen durch die Haube 61 hindurch auf die Haut des Patienten 7 aufbringbar ist. Dies kann, wie in dem in **Fig. 7** dargestellten Ausführungsbeispiel dadurch geschehen, dass sich die Ausnehmungen 62 der Haube 61 mit den Ausnehmungen 3 im Grundkörper 1 der jeweiligen Elektrodenanordnung 10 decken.

Es ist jedoch auch alternativ möglich, dass die einzelnen Elektrodenanordnungen 10 jeweils in eine eigene Ausnehmung 62 der Haube 61 eingebracht sind und die Haube 61 im Bereich ihrer Ausnehmung 62 fortsetzen. Der äußere Rand der Elektrodenanordnung 10 schließt in diesem Fall an den Innenrand einer der Ausnehmungen 62 der Haube 61 an.

In **Fig. 8** ist ein Teil einer weiteren, als Manschette 63 ausgebildeten Ausführungsform der Erfindung im Betrieb mit einem Patienten 7 dargestellt. Die Manschette 63 weist, wie auch die in **Fig. 7** dargestellte Haube 61 eine Anzahl von an ihrer Innenfläche angeordneten Elektrodenanordnungen 10 auf. Die Ausnehmungen 62 der Manschette 63 sind mit den Ausnehmungen 3 im Grundkörper 1 der Elektrodenanordnung 10 zur Deckung gebracht, sodass ein Gel 5 auf die Hautoberfläche 4 des Patienten 7 aufgebracht werden kann. Das Gel 5 befindet sich nach dem Einbringen durch die Ausnehmung 3 im schraffiert dargestellten und von Haaren gefüllten Zwischenbereich, im folgenden Haarbereich 72 genannt, zwischen der Hautoberfläche 4 und dem Grundkörper 1. Der Grundkörper 1 und die Hautoberfläche 4 des Patienten 7 werden einerseits durch die Haare, andererseits durch die Stiftelektroden 2 auf Abstand gehalten. Das Gel 5 diffundiert in den Haarbereich 72 und gelangt auf die Auflagepunkte 8 der Stiftelektroden 2 an der Hautoberfläche 4, wodurch der Kontaktwiderstand deutlich herabgesetzt wird.

Für den Fall, dass bereits ein ausreichender elektrischer Kontakt erzielt worden ist und Signale von für die jeweilige Untersuchung ausreichender Qualität zur Verfügung stehen, kann die Beaufschlagung der Hautoberfläche 4 sowie des Haarbereichs 72 mit Gel 5 unterblieben.

Alternativ zu Gel 5 kann auch ein wässriger Schaum oder ein in Wasser getränkter Schwamm (beides nicht dargestellt) verwendet werden, der in der Ausnehmung 3 des Grundkörpers 1 und zwischen den Stiftelektroden 2 angeordnet ist.

Um ein Eindringen von Feuchtigkeit oder Wasser in die Elektrodenanordnung 10 zu verhindern, können die elektrisch aktiven Komponenten der Elektrodenanordnung 10 wie etwa die Verstärkeranordnung 13 wasserdicht ausgebildet sein. Das Ausgangssignal des Verstärkers 13 wird über ein in den Fig. 5 bis 8 nicht dargestelltes Kabel an eine Datenverarbeitungseinheit weitergeleitet.

Alternativ kann vorgesehen sein, dass die Stiftelektroden 2 nicht vollständig aus Gold und/oder TiN und/oder IrO₂ oder einer Legierung mit Gold und/oder TiN und/oder IrO₂ bestehen sondern aus einem Trägerkörper, beispielsweise aus Metall, bestehen und lediglich mit einer der genannten hoch leitfähigen Legierungen beschichtet sind.

## Patentansprüche

1. Vorrichtung mit einer Anzahl von Elektrodenanordnungen (10), die an die Hautoberfläche (4) eines tierischen oder menschlichen Lebewesens anlegbar sind und mit denen Spannungen und Ströme von der Hautoberfläche (4) abgreifbar sind und mit einer mit einem flächigem oder folienartigem Formteil gebildeten flexiblen, insbesondere dehnbaren, Haube (6) wobei die Elektrodenanordnungen (10) einen Grundkörper (1) sowie einer Anzahl von vom Grundkörper (1) nach derselben Richtung abstehenden Stiftelektroden (2) aufweisen und die Elektrodenanordnungen (10) mit dem Halteelement (6) befestigt bzw. verbunden sind und der Grundkörper (1) der jeweiligen Elektrodenanordnung (10) mit dem Halteelement (6) verbunden ist, wobei die Stiftelektroden (2) sämtlicher Elektrodenanordnungen (10) nach derselben Richtung abstehen, **dadurch gekennzeichnet, dass** die Stiftelektroden (2) auf einem gemeinsamen Elektrodenring (14) angeordnet und mit diesem elektrisch leitend verbunden sind, wobei der Elektroderring (14) vorzugsweise aus demselben Material besteht wie die Stiftelektroden (2).

2. Vorrichtung nach Anspruch 1, **dadurch gekennzeichnet, dass** im Grundkörper (1) bei einer Elektrodenanordnung (10), insbesondere bei allen Elektrodenanordnungen (10), eine durchgängige Ausnehmung (3) vorgesehen ist, durch die bei Anliegen der Stiftelektroden (2) an der Hautoberfläche (4) ein den Kontaktwiderstand zwischen der Hautoberfläche (4) und den Stiftelektroden (2) verringerndes Gel (5) auf die Hautoberfläche (4) aufbringbar ist.

3. Vorrichtung nach Anspruch 2, **dadurch gekennzeichnet, dass** das Halteelement (5) Ausnehmungen (62) aufweist, deren jeweilige Position an die Position jeweils einer der Ausnehmungen (3) im Grundkörper (1) angepasst ist, sodass Gel (5) durch die Ausnehmungen (62) im Halteelement (6) und durch die Ausnehmung (3) Im Grundkörper (1) auf die Hautoberfläche (4) aufbringbar ist

4. Vorrichtung nach einem der Ansprüche 2 oder 3, **dadurch gekennzeichnet, dass** die Grundkörper (1) der Elektrodenanordnungen (10) flach oder plattenförmig mit zwei einander gegenüberliegenden Oberflächenbereichen (11, 12) ausgebildet sind, wobei die in den Grundkörpern (1) ausgebildeten Ausnehmungen (3) durchgängig ausgebildet sind und die jeweiligen Berandungen (31) der Ausnehmungen (3) jeweils die beiden Oberflächenbereiche (11, 12) der Grundkörper (1) miteinander verbindet.

5. Vorrichtung nach einem der Ansprüche 1 bis 4, **dadurch gekennzeichnet, dass** die Stiftelektroden (2) am Rand der Ausnehmung (3) an dem Grundkörper (1) befestigt sind.

6. Vorrichtung nach einem der Ansprüche 1 bis 5, **dadurch gekennzeichnet, dass** die Ausnehmung (3) der Elektrodenanordnung (10) kreisförmig ausgebildet sind.

7. Vorrichtung nach einem der Ansprüche 1 bis 6, **dadurch gekennzeichnet, dass** je zwei benachbart angeordnete Stiftelektroden (2) einer Elektrodenanordnung (10) jeweils voneinander denselben Abstand haben.

8. Vorrichtung nach einem der vorangehenden Ansprüche, **dadurch gekennzeichnet, dass** die Stiftelektroden (2) eine Anzahl, Anordnung und Höhe oder Länge aufweisen, sodass sie eine auf der Hautoberfläche (4) befindliche Behaarung durchdringen können, jedoch eine stabile Auflage gewährleisten, und/oder dass die Höhe der Stiftelektroden (2) zwischen 1 mm und 30 mm liegt.

9. Vorrichtung nach einem der Ansprüche 2 bis 8, **dadurch gekennzeichnet, dass** die von der jeweiligen Ausnehmung (3) der Elektrodenanordnungen (10) ausgenommene Fläche des Grundkörpers (1) 0,785 mm² bis 700 mm² beträgt und/oder der Radius der jeweiligen Ausnehmung (3) zwischen 1 mm und 30 mm beträgt.

10. Vorrichtung nach einem der vorangehenden Ansprüche, **dadurch gekennzeichnet, dass** der Durchmesser der Stiftelektroden (2) derart gewählt ist, dass die Stiftelektrode (2) durch die Behaarung der Hautoberfläche (4) hindurchdringen kann, bei einem elektrischen Kontakt ermöglichenden Anpressdruck die Hautoberfläche (4) jedoch nicht verletzt, und/oder dass der Durchmesser der Stiftelektroden (2) zwischen 0,5 mm und 5 mm liegt.

11. Vorrichtung nach einem der Ansprüche 3 bis 10, **dadurch gekennzeichnet, dass** das dem Grundkörper (1) ferne Ende der Stiftelektroden (2) halbkugelförmig ausgebildet ist.

12. Vorrichtung nach einem der vorangehenden Ansprüche, **dadurch gekennzeichnet, dass** die Stiftelektroden (2) hochleitfähig sind und insbesondere aus Gold, TiN oder einer Gold und/oder TiN und/oder, IrO₂ enthaltenden Legierung bestehen.

13. Vorrichtung nach einem der vorangehenden Ansprüche, **dadurch gekennzeichnet, dass**, insbesondere auf dem Grundkörper (1) der jeweiligen Elektrodenanordnung (10), eine Verstärkeranordnung (13), insbesondere in einem Abstand von weniger als 20 mm zur nächstgelegenen Stiftelektrode (2), vorgesehen oder angeordnet ist und die Stiftelektroden (2) an die Verstärkeranordnung (13) angeschlossen sind.

14. Vorrichtung nach Anspruch 13, **dadurch gekennzeichnet, dass** die Verstärkeranordnung (13) auf einem mit dem jeweiligen Grundkörper (1) elektrisch und mechanisch, insbesondere über Rastverbindungen (16, 19, 19a), lösbar verbindbaren separaten weiteren Grundkörper (15) angeordnet ist.

15. Vorrichtung nach Anspruch 14, **dadurch gekennzeichnet, dass** das Halteelement (6) eine Anzahl von Ausnehmungen (62) aufweist und die Rastverbindungen (19, 19a) die Ausnehmungen (62) des Halteelements (6) durchsetzen.

## Claims

1. Device having a number of electrode arrangements (10) that can be applied to the surface (4) of the skin of a living animal or human, and with which voltages and currents from the skin surface (4) can be obtained, and having a flexible, in particular elastic, hood (6) consisting of a flat or film-like moulded part, whereby the electrode arrangements (10) have a base (1) and a number of pin electrodes (2) standing out from the base (1) in the same direction, and the electrode arrangements (10) are attached or connected with the retaining element (6), and the base (1) of the respective electrode arrangement (10) is connected with the retaining element (6), whereby the pin electrodes (2) of all electrode arrangements (10) stand out in the same direction, **characterised in that** the pin electrodes (2) are arranged on a shared electrode ring (14) and are conductively connected therewith, and whereby the electrode ring (14) preferably consists of the same material as the pin electrodes (2).

2. Device according to claim 1, **characterised in that**, in the base (1) of an electrode arrangement (10), in particular all electrode arrangements (10), a continuous recess (3) is provided through which a gel (5) reducing the contact resistance between the skin surface (4) and the pin electrodes (2) can be applied to the skin surface (4) when the pin electrodes (2) are applied to the skin surface (4).

3. Device according to claim 2, **characterised in that** the retaining element (6) has recesses (62), the respective position of which is adapted to the respective position of one of the recesses (3) in the base (1), such that gel (5) can be applied to the skin surface (4) through the recesses (62) in the retaining element (6) and through the recess (3) in the base (1).

4. Device according to either of claims 2 or 3, **characterised in that** the bases (1) of the electrode arrangements (10) are flat or plate-shaped and have two opposite surface areas (11, 12), whereby the recesses (3) formed in the bases (1) are continuous and the respective boundaries (31) of the recesses (3) connect the two surfaces areas (11, 12) of the bases (1).

5. Device according to any of claims 1 - 4, **characterised in that** the pin electrodes (2) are attached to the base (1) on the edge of the recess (3).

6. Device according to any of claims 1 - 5, **characterised in that** the recesses (3) of the electrode arrangement (10) are circular..

7. Device according to any of claims 1 - 6, **characterised in that** two respective adjacent pin electrodes (2) of an electrode arrangement (10) are each at the same distance from one another.

8. Device according to any of the foregoing claims, **characterised in that** the pin electrodes (2) have a number, arrangement, and height or length that is such that they can penetrate any hair present on the skin surface (4), but provide a stable position, and/or that the height of the pin electrodes (2) is between 1 mm and 30 mm.

9. Device according to any of claims 2 - 8, **characterised in that** the area of the base (1) recessed from the respective recess (3) of the electrode arrangements (10) is 0.785 - 700 mm2, and/or the radius of the respective recess (3) is between 1 and 30 mm.

10. Device according to any of the foregoing claims, **characterised in that** the diameter of the pin electrodes (2) is such that the pin electrodes (2) can penetrate the hair on the skin surface (4), but does not injure the skin surface (4) at a contact pressure allowing for electrical contact, and/or that the diameter of the pin electrodes (2) is between 0.5 and 5 mm.

11. Device according to any of claims 3 - 10, **characterised in that** the end of the pin electrodes (2) farthest from the base (1) is semicircular.

12. Device according to any of the foregoing claims, **characterised in that** the pin electrodes (2) are highly conductive, and, in particular, consist of gold, TiN oder an alloy containing gold and/or TiN and/or IrO2.

13. Device according to any of the foregoing claims, **characterised in that**, in particular on the base (1) of the respective electrode arrangement (10), an amplifier arrangement (13) is provided or arranged, in particular at a distance of less than 20 mm from the nearest pin electrode (2), and the pin electrodes (2) are connected to the amplifier arrangement (13).

14. Device according to claim 13, **characterised in that** the amplifier arrangement (13) is arranged on an additional, separate base (15) that can be removably electrically and mechanically connected with the respective base (1), in particular via click-on connections (16, 19, 19a).

15. Device according to claim 14, **characterised in that** the retaining element (6) has a number of recesses (62), and that the click-on connections (19, 19a) pass through the recesses (62) of the retaining element (6).

## Revendications

1. Dispositif comportant un certain nombre de systèmes d'électrodes (10), qui peuvent être appliqués sur la surface (4) de la peau d'un être vivant animal ou humain, et à l'aide desquels des tensions et des courants peuvent être prélevés de la surface (4) de la peau, ainsi qu'un capot (6), flexible, en particulier extensible, formé à partir d'une pièce moulée bidimensionnelle ou de type feuille, les systèmes d'électrodes (10) comportant un corps de base (1) ainsi qu'un certain nombre d'électrodes-barres (2), en saillie par rapport au corps de base (1), dans la même direction, et les systèmes d'électrodes (10) étant fixés ou raccordés à l'élément d'arrêt (6), et le corps de base (1) de chaque système d'électrodes (10) étant relié à l'élément d'arrêt (6), les électrodes-barres (2) de l'ensemble des systèmes d'électrodes (10) dépassant dans la même direction, **caractérisé en ce que** les électrodes-barres (2) sont disposées sur un anneau porte-électrodes (14) commun, et sont reliées à ce dernier d'une manière conductrice de l'électricité, l'anneau porte-électrode (14) étant de préférence constitué du même matériau que les électrodes-barres (2).

2. Dispositif selon la revendication 1, **caractérisé en ce qu'**est prévu dans le corps de base (1), dans un système d'électrodes (10), en particulier dans tous les systèmes d'électrodes (10), un évidement continu (3), à travers lequel, lors de l'application des électrodes-barres (2) sur la surface (4) de la peau, un gel (5), diminuant la résistance de contact entre la surface (4) de la peau et les électrodes-barres (2), peut être appliqué sur la surface (4) de la peau.

3. Dispositif selon la revendication 2, **caractérisé en ce que** l'élément d'arrêt (6) comprend des évidements (62), la position de chacun d'eux étant adaptée à la position de chacun des évidements (3) aménagés dans le corps de base (1) de telle sorte que le gel (5) puisse être appliqué sur la surface (4) de la peau à travers les évidements (62) aménagés dans l'élément d'arrêt (6) et l'évidement (3) aménagé dans le corps de base (1).

4. Dispositif selon l'une des revendications 2 ou 3, **caractérisé en ce que** le corps de base (1) des systèmes d'électrodes (10) est configuré plat ou en forme de plaque, avec deux zones superficielles opposées l'une à l'autre (11, 12), les évidements (3) aménagés dans les corps de base (1) ayant une configuration continue, et les bordures (31) de chacun des évidements (3) reliant l'une à l'autre les deux zones superficielles (11, 12) des corps de base (1).

5. Dispositif selon l'une des revendications 1 à 4, **caractérisé en ce que** les électrodes-barres (2) sont fixées, au niveau du bord de l'évidement (3), au corps de base (1).

6. Dispositif selon l'une des revendications 1 à 5, **caractérisé en ce que** l'évidement (3) du système d'électrodes (10) a une configuration circulaire.

7. Dispositif selon l'une des revendications 1 à 6, **caractérisé en ce que** les électrodes-barres de chaque groupe de deux électrodes-barres (2) disposées au voisinage l'une de l'autre d'un système d'électrodes (10) ont toujours entre elles la même distance.

8. Dispositif selon l'une des revendications précédentes, **caractérisé en ce que** les électrodes-barres (2) sont présentes selon un nombre, un arrangement, et une hauteur ou une longueur, tels qu'elles puissent traverser un pelage se trouvant sur la surface (4) de la peau, tout en garantissant un positionnement stable, et/ou que la hauteur des électrodes-barres (2) soit comprise entre 1 et 30 mm.

9. Dispositif selon l'une des revendications 2 à 8, **caractérisé en ce que** l'aire du corps de base (1), à l'exception de l'évidement (3) de chacun des systèmes d'électrodes (10), est de 0,785 à 700 mm², et/ou que le rayon de chaque évidement (3) est compris entre 1 et 30 mm.

10. Dispositif selon l'une des revendications précédentes, **caractérisé en ce que** le diamètre des électrodes-barres (2) est choisi de telle sorte que l'électrode-barre (2) puisse passer à travers le pelage de la surface (4) de la peau, mais cependant sans endommager la surface (4) de la peau dans le cas d'une pression d'appui autorisant un contact électrique, et/ou que le diamètre des électrodes-barres (2) est compris entre 0,5 et 5 mm.

11. Dispositif selon l'une des revendications 3 à 10, **caractérisé en ce que** l'extrémité des électrodes-barres (2) éloignées du corps de base (1) a une configuration hémisphérique.

12. Dispositif selon l'une des revendications précédentes, **caractérisé en ce que** les électrodes-barres (2) sont très conductrices, et sont constituées en particulier d'or, de TiN ou d'un alliage contenant de l'or et/ou du TiN et/ou de l'IrO₂.

13. Dispositif selon l'une des revendications précédentes, **caractérisé en ce que**, en particulier sur le corps de base (1) de chaque système d'électrodes (10), un arrangement de renforcement (13) est prévu ou disposé en particulier à une distance inférieure à 20 mm de l'électrode-barre (2) la plus proche, et que les électrodes-barres (2) sont connectées à l'arrangement de renforcement (13).

14. Dispositif selon la revendication 13, **caractérisé en ce que** l'arrangement de renforcement (13) est disposé sur un corps de base (15) supplémentaire, distinct, pouvant être électriquement et mécaniquement raccordé d'une manière amovible, en particulier par des liaisons avec encliquetage (16, 19, 19a), au corps de base correspondant (1).

15. Dispositif selon la revendication 14, **caractérisé en ce que** l'élément d'arrêt (6) comprend un certain nombre d'évidements (62), et que les liaisons avec encliquetage (19, 19a) traversent les évidements (62) aménagés dans l'élément d'arrêt (6).
